# EUROPÄISCHE PATENTSCHRIFT

(11) **EP 3 628 356 B1**
(45) Veröffentlichungstag und Bekanntmachung des Hinweises auf die Patenterteilung: **22.12.2021**
(21) Anmeldenummer: 18196941.1
(22) Anmeldetag: 26.09.2018
(51) Int. Cl.: A61M 11/02, A61M 11/06, A61M 35/00, B05B 11/00, A61M 13/00, B05B 7/24, B05B 7/26, B05B 9/047

(54) **MEDIZINISCHES INSTRUMENT UND ERZEUGUNGSEINRICHTUNG**
MEDICAL INSTRUMENT AND CREATION DEVICE
INSTRUMENT MÉDICAL ET DISPOSITIF DE GÉNÉRATION

(43) Veröffentlichungstag der Anmeldung: 01.04.2020
(73) Patentinhaber: Erbe Elektromedizin GmbH, 72072 Tübingen (DE)
(72) Erfinder: NEAGOS, Alexander, 72770 Reutlingen (DE); ENDERLE, Markus, 72070 Tübingen (DE); SCHÄFER, Christian, 72127 Kusterdingen (DE)
(74) Vertreter: Rüger Abel Patentanwälte PartGmbB

(56) Entgegenhaltungen:
- EP-A1- 2 338 587
- EP-B1- 2 338 587
- US-A- 4 376 376
- US-A1- 2003 181 917
- US-A1- 2008 125 742
- US-A1- 2009 326 621
- US-A1- 2011 230 820
- US-B2- 8 945 605

## Beschreibung

Die Erfindung betrifft ein medizinisches Instrument zur Anwendung eines Aerosols sowie eine Erzeugungseinrichtung für ein Aerosol.

Aus WO 2011/029572 A1 sowie WO 2011/029 573 A1 sind medizinische Instrumente bekannt, welche einen Kopf am distalen Ende des Instruments aufweisen, in welchem eine Flüssigkeit, insbesondere Kochsalzlösung, und ein Gas, beispielsweise CO2, zusammengeführt werden, um mit dem Instrument ein Gemisch aus in dem Gas feinverteilten Flüssigkeitströpfchen (Aerosol) abzugeben.

EP 0 740 926 A2 beschreibt eine Vorrichtung mit einer Aerosolstrahlbildungskomponente.

Nachfolgende Dokumente offenbaren weitere bekannte Vorrichtungen mit Aerosolstrahlkomponenten: US 2011/230820, US 8 945 605, US 2008/125742, US 2003/181917, US 4 376 376, US 2009/326621, EP 2 338 587.

Medizinische Instrumente, insbesondere deren Köpfe, am distalen Ende der medizinischen Instrumente, bzw. Applikatoren sind oftmals nur zum einmaligen Gebrauch gedacht, da diese sauber und steril sein müssen.

Aufgabe der vorliegenden Erfindung ist es, ein verbessertes Konzept für ein Instrument zu Abgabe von Aerosol anzugeben.

Diese Aufgabe wird mit einer Einrichtung nach Anspruch 1 gelöst.

Das erfindungsgemäße medizinische Instrument weist einen Aerosolauslass zur Abgabe von Aerosol mittels des medizinischen Instruments auf. Das Instrument ist mittels einer Aerosolleitung, besonders bevorzugt einer Aerosolschlauchleitung (im Folgenden auch Schlauchleitung genannt), fluidisch mit einer Erzeugungseinrichtung verbunden, welche Erzeugungseinrichtung dazu eingerichtet ist, Aerosol zu erzeugen und die Aerosolleitung mit Aerosol zur Abgabe von Aerosol mittels des medizinischen Instruments zu speisen. Die Erzeugungseinrichtung ist dazu eingerichtet, das Aerosol durch Zerstäubung einer Flüssigkeit mittels eines Gasstroms zu erzeugen.

Ausführungsformen des erfindungsgemäßen medizinischen Instruments können beispielsweise Instrumente für die offene Chirurgie oder die endoskopische, insbesondere laparoskopische, Chirurgie sein.

In Ausführungsformen weist das Instrument einen Handhabungsabschnitt auf, welcher Handhabungsabschnitt den Aerosolauslass aufweist, wobei der Handhabungsabschnitt mittels der Aerosolleitung, beispielsweise einer Aerosolschlauchleitung, mit der Erzeugungseinrichtung fluidisch verbunden ist.

Der Handhabungsabschnitt kann beispielsweise durch einen Applikator oder ein Handgriffteil gebildet werden. Der Handhabungsabschnitt ist dazu eingerichtet, das medizinische Instrument während des Eingriffs, welcher mit Hilfe des medizinischen Instruments erfolgt, zu führen, zu bedienen und/oder beispielsweise den Aerosolauslass für die gewünschte Abgaberichtung des Aerosols auszurichten.

Das medizinische Instrument kann beispielsweise eine Sonde zur Abgabe von Aerosol sein, welche Sonde dazu eingerichtet sein kann, in dem Arbeitskanal eines Endoskops angeordnet zu sein, so dass das distale Ende der Sonde am distalen Ende des Endoskops aus dem Arbeitskanal herausschaut. Der Auslass kann am distalen Ende der Sonde angeordnet sein.

Das medizinische Instrument ist vorzugsweise lösbar an die Erzeugungseinrichtung angeschlossen, um das medizinische Instrument nach der Verwendung von der Erzeugungseinrichtung für das Aerosol abzukoppeln, um beispielsweise ein unbenutztes medizinisches Instrument mit der Erzeugungseinrichtung zu verbinden.

Das medizinische Instrument ist insofern vereinfacht, als dass es keiner separaten Gas- und Flüssigkeitsleitungen bedarf, um Gas und Flüssigkeit unabhängig voneinander bis zu einer Aerosolerzeugungseinheit im Instrument, beispielsweise im Handhabungsabschnitt des Instruments, zu leiten. Es genügt eine, vorzugsweise flexible, Aerosolleitung von der Erzeugungseinrichtung bis **zu dem Instrument,** in Ausführungsformen bis zu dem Handhabungsabschnitt. Selbstverständlich kann das medizinische Instrument weitere Leitungen, auch Gas- oder Flüssigkeitsleitungen und/oder elektrische Leitungen, aufweisen, welche zu dem Instrument, in Ausführungsformen zu einem Handhabungsabschnitt, führen können, welche aber vorzugsweise nicht der Aerosolerzeugung in dem medizinischen Instrument, z.B. in einem Handhabungsabschnitt des medizinischen Instruments, dienen.

Gegenüber einem Instrument mit zwei gesonderten Leitungen zu einer Mischeinheit zur Erzeugung des Aerosols in dem Instrument, z.B. in oder an einem Handhabungsabschnitt des Instruments, können die Entwicklungs- und Fertigungskosten reduziert sein, wenn eine Erzeugungseinrichtung zur Erzeugung von Aerosol in dem Instrument, z.B. in oder an einem Handhabungsabschnitt des Instruments, fehlt. Entwicklungs- und Fertigungskosten sollten bei Produkten zur einmaligen Verwendung besonders niedrig sein. Dies macht das medizinische Instrument besonders kostengünstig in der Entwicklung und Fertigung. Bei dem medizinischen Instrument kann es sich um ein Instrument zur einmaligen Verwendung handeln.

Mit der Aerosolleitung lassen sich bevorzugt große Abstände zwischen der Erzeugungseinrichtung für das Aerosol und dem Instrument, in Ausführungsformen zwischen der Erzeugungseinrichtung für das Aerosol und dem Handhabungsabschnitt, bzw. zwischen der Erzeugungseinrichtung und dem Aerosolauslass realisieren. Die Länge der Aerosolleitung zwischen der Erzeugungseinrichtung und dem Aerosolauslass oder dem Instrument beträgt vorzugsweise wenigstens 1m, besonders bevorzugt wenigstens 2m.

Die Erzeugungseinrichtung stützt sich bei Anwendung des Instruments vorzugsweise am Untergrund ab, beispielsweise über eine Tragvorrichtung. Die Erzeugungseinrichtung kann beispielsweise von einer Tragvorrichtung getragen werden, welche sich am Untergrund abstützt. In solchen Ausführungsformen muss der Benutzer des Instruments das Gewicht der Erzeugungseinrichtung bei der medizinischen Anwendung des Instruments nicht selber tragen. Die Erzeugungseinrichtung kann beispielsweise an einem Gerät angeordnet sein, welches eine elektrische HF-Leistung zur Beaufschlagung einer Elektrode bereitstellt, welche Elektrode in Ausführungsformen des erfindungsgemäßen Instruments in oder an dem Instrument, beispielsweise in oder an einem Handhabungsabschnitt, angeordnet sein kann. Das Gerät kann sich am Untergrund, beispielsweise über die Tragvorrichtung, abstützen.

Das Instrument oder der Handhabungsabschnitt, sofern vorhanden, ist bevorzugt relativ zu der Erzeugungseinrichtung beweglich. Die Erzeugungseinrichtung kann von dem Instrument oder dem Handhabungsabschnitt, sofern vorhanden, derart entkoppelt sein, dass die Erzeugungseinrichtung nicht auf Grund der Bewegung des Instruments bei der medizinischen Anwendung zwingend mitbewegt wird. Die Erzeugungseinrichtung ist vorzugsweise an einem proximalen Ende einer Vorrichtung angeordnet, welche das erfindungsgemäße Instrument aufweist. Alternativ oder zusätzlich ist der Handhabungsabschnitt am distalen Ende der Vorrichtung angeordnet, welche das erfindungsgemäße Instrument aufweist.

Besonders kostengünstig sowie besonders leise und einfach zu bedienen wird eine Vorrichtung mit dem Instrument, wenn die Flüssigkeit ohne elektrisch betriebene Pumpe zu der Erzeugungseinrichtung gefördert wird. Auf eine aktive Fördereinheit für die Flüssigkeit, wie z.B. eine Spritzenpumpe oder eine Rollenpumpe kann verzichtet werden, wenn eine passive Förderung bzw. Zumischung der Flüssigkeit durch Drücken der Flüssigkeit zu der Mischeinheit durch Druck oberhalb von Atmosphärendruck und/oder durch Saugen basierend auf dem Venturi- oder Strahlpumpenprinzip erfolgt.

Erfindungsgemäß wird eine Erzeugungseinrichtung angegeben, mit welcher sich ein Aerosol erzeugen lässt. Die Erzeugungseinrichtung ist erfindungsgemäß in einer Vorrichtung mit einem erfindungsgemäßen Instrument **zu verwenden, welches erfindungsgemäße** Instrument hierin beschrieben ist. Die Erzeugungseinrichtung lässt sich jedoch auch unabhängig von den hier beschriebenen Ausführungsformen des erfindungsgemäßen Instruments verwenden. Die erfindungsgemäße Erzeugungseinrichtung kann zur Beaufschlagung eines Instruments mit Aerosol beispielsweise für die offene Chirurgie oder die endoskopische, insbesondere laparoskopische, Chirurgie verwendet werden, um mittels des Instruments Aerosol abgeben zu können.

Die Erzeugungseinrichtung weist eine Mischeinheit auf, um zur Erzeugung eines Aerosols Flüssigkeit und Gas miteinander zu mischen. Die Mischeinheit ist an einen Flüssigkeitsbehälter zur Versorgung der Mischeinheit mit Flüssigkeit angeschlossen. Die Erzeugungseinrichtung ist dazu eingerichtet, den Gasdruck eines unter Druck oberhalb Atmosphärendruck stehenden Gases (Druckgas) zu nutzen, die Flüssigkeit mit Druck zu beaufschlagen, um die Flüssigkeit aus dem Flüssigkeitsbehälter zu der Mischeinheit zu drücken. Das Gas kann dabei unmittelbar auf die Flüssigkeitsoberfläche drücken. Alternativ kann das Gas auf einen Behälter, beispielsweise einen Beutel, drücken, der die Flüssigkeit enthält. Das Gas kann gegen einen Kolben oder eine Membran drücken, welcher bzw. welche ihrerseits auf die Flüssigkeit direkt oder einen Flüssigkeitsbehälter, beispielsweise Beutel, drückt.

Ausführungsformen der erfindungsgemäßen Erzeugungseinrichtung, in welchen ein komprimierbarer Flüssigkeitsbehälter, z.B. ein Beutel, der bis auf einen Anschluss zum Anschließen an die Mischeinheit geschlossen ist, in einem Druckbehälter angeordnet ist, bieten den Vorteil, dass sichergestellt werden kann, dass die Flüssigkeitsversorgung unabhängig von etwa der Ausrichtung des Flüssigkeitsbehälters, beispielsweise des Beutels, bzw. des Druckbehälters ist, welcher den Flüssigkeitsbehälter enthält.

Die Flüssigkeitsversorgung ist bevorzugt unabhängig von der Position des Flüssigkeitsbehälters. Dies wird bevorzugt dadurch erreicht, dass an dem Anschluss der Mischeinheit zum Zuführen von Flüssigkeit unabhängig von der Ausrichtung und Position des Flüssigkeitsbehälters stets ein Mindestflüssigkeitsdruck ansteht. Dies kann durch das Beaufschlagen der Flüssigkeit mit Druck erreicht werden.

Die Vorrichtung ist besonders einfach, wenn die Erzeugungseinrichtung an einen stationären oder mobilen Druckgasspeicher, wie beispielsweise eine Gasflasche, oder ein stationäres Druckgasnetz, als Quelle für das unter Druck stehende Gas angeschlossen ist. Als Gas kann beispielsweise CO2 verwendet werden. Die Quelle ist bevorzugt dazu eingerichtet, Gas mit einem Druck von einigen Bar bereitzustellen.

Wenn das zum Erzeugen des Aerosols mit Flüssigkeit zu mischende Gas und das Gas, welches dazu bestimmt ist, auf die Flüssigkeit zu drücken, aus derselben Gasquelle stammt, müssen keine zwei unabhängigen Gasquellen (z.B. zwei Gasflaschen) bereitgestellt werden. Vielmehr reicht eine Gasquelle (z.B. eine Gasflasche) aus, welche Gas sowohl zur Speisung der Mischeinheit als auch zum Beaufschlagen der Flüssigkeit in dem Flüssigkeitsbehälter mit Druck liefert.

In Ausführungsformen wird ein Flüssigkeitsbehälter, welcher die Flüssigkeit enthält, von außen mit Druck beaufschlagt, um die Flüssigkeit zu der Mischeinheit zu fördern.

Der Flüssigkeitsbehälter ist vorzugsweise dazu eingerichtet und bestimmt komprimiert zu werden, um den Flüssigkeitsbehälter aufgrund des Druckes komprimieren zu können, um somit die Flüssigkeit aus dem Flüssigkeitsbehälter zu der Mischeinheit zu drücken.

Der Flüssigkeitsbehälter kann beispielsweise ein Beutel sein. Bevorzugt wird ein in der Klinik, in welcher die Erzeugungseinrichtung verwendet wird, standardmäßig verfügbarer Beutel verwendet. Wird ein Standardbeutel verwendet, kann ein Befüllen eines speziellen Flüssigkeitsbehälters für die Vorrichtung entfallen. Bei der Flüssigkeit kann es sich beispielsweise um sterile isotonische Kochsalzlösung handeln. Der Beutel kann beispielsweise ein Infusionsbeutel sein, wie er standardmäßig in der Klinik verwendet wird.

In Ausführungsformen wird der Flüssigkeitsbehälter in einem Druckbehälter angeordnet, wobei der Druckbehälter mit Druckgas beaufschlagt wird, um die Flüssigkeit aus dem Flüssigkeitsbehälter zu der Mischeinheit zu drücken. In dem Druckbehälter kann der Gasdruck des Gases unmittelbar auf den Flüssigkeitsbehälter wirken. Dies macht die Erzeugungseinrichtung besonders einfach. Alternativ kann der Druck auf beispielsweise eine Membran oder einen Kolben wirken, welche Membran oder welcher Kolben auf den Flüssigkeitsbehälter wirkt. Oder die Flüssigkeit ist in einem Zylinder in einem von zwei Arbeitsräumen angeordnet, welche von einem Kolben voneinander getrennt sind, wobei der andere Arbeitsraum mit dem Druckgas beaufschlagt wird, um die Flüssigkeit mittels des Kolbens aus dem einen Arbeitsraum herauszudrücken.

In Ausführungsformen kann der Druckbehälter selbst den Flüssigkeitsbehälter bilden, indem die Wandung des Druckbehälters die Flüssigkeit am Auslaufen hindert. Der Raum über dem Flüssigkeitsspiegel kann mit unter Druck stehendem Gas beaufschlagt werden, welches Kontakt mit der Flüssigkeitsoberfläche haben kann, um die Flüssigkeit aus dem Druckbehälter zu drücken.

Die Mischeinheit arbeitet vorzugsweise nach dem Venturi- oder Strahlpumpenprinzip, um die Flüssigkeit mit dem Gas zu mischen. Die Mischeinheit weist vorzugsweise einen Kanal mit einer Verengung auf, wobei der Kanal stromaufwärts der Verengung an die Druckquelle angeschlossen ist, und wobei der Flüssigkeitsbehälter stromabwärts der Verengung und/oder an der Verengung an den Kanal angeschlossen ist. Wird der Kanal stromaufwärts der Verengung mit Druckgas gespeist, so kommt es aufgrund der Verengung zu einer Beschleunigung des Gasstroms und zu einer Verminderung des statischen Drucks in der Verengung und stromabwärts der Verengung. Wenn eine Flüssigkeitsleitung vom Flüssigkeitsbehälter in dem Bereich mit vermindertem statischem Druck mit der Mischeinheit verbunden ist, so fördert der verminderte statische Druck das Beaufschlagen des Kanals mit Flüssigkeit, welche von dem beschleunigten Gas mitgerissen wird, um ein Aerosol zu erzeugen.

Ein besonders einfacher Aufbau wird erhalten, wenn der Kanal stromaufwärts der Verengung an dieselbe Druckquelle für unter Druck stehendes Gas angeschlossen ist, welche Druckquelle auch dazu dient, die Flüssigkeit in dem Flüssigkeitsbehälter mit Druck zu beaufschlagen, um diese zu der Mischeinheit zu drücken. Insbesondere kann der Kanal an einen Druckgasspeicher (stationär oder mobil), wie beispielsweise eine Gasflasche, oder ein Druckgasnetz, beispielsweise ein Druckgasnetz eines Gebäudes, insbesondere Klinikgebäudes, angeschlossen sein, wobei der Druckgasspeicher oder das Druckgasnetz auch dazu dient, die Flüssigkeit in dem Flüssigkeitsbehälter mit Druck zu beaufschlagen. Beispielsweise kann zwischen der Verengung des Kanals und der Druckquelle eine Leitungsverzweigung mit zwei Zweigleitungen vorgesehen sein, welche eine Zweigleitung zu der Verengung führt und welche andere Zweigleitung zu dem Druckbehälter führt. Wenn die Erzeugungseinrichtung eine an die Druckquelle angeschlossene Leitung aufweist, welche zwischen der Verengung und der Druckquelle verzweigt, um aus der Leitung sowohl die Verengung mit Gas zu speisen als auch das Gas zum Beaufschlagen der Flüssigkeit mit Druck bereitzustellen, so ist die Verzweigung vorzugsweise in der Mischeinheit angeordnet. Der Gasstrom aus der Druckquelle wird in zwei Teilströme aufgeteilt, von denen ein erster Teilstrom zu der Mischeinheit strömt und ein zweiter Teilstrom zu dem Flüssigkeitsbehälter strömt, um die Flüssigkeit mittels des zweiten Teilstroms mit Druck zu beaufschlagen.

In einer Vorrichtung ist ein Instrument zum Abgeben von Aerosol über eine Aerosolleitung an eine Erzeugungseinheit, wie sie hierin beschrieben ist, angeschlossen, wobei das proximale Ende der Aerosolleitung und die Erzeugungseinrichtung beim Anwenden des Instruments außerhalb des Körpers des Patienten verbleibt. Das proximale Ende der Aerosolleitung ist an die hierin beschriebene Erzeugungseinrichtung angeschlossen, um das Instrument mittels der Erzeugungseinrichtung über die Aerosolleitung mit Aerosol zu speisen.

Weitere Merkmale des erfindungsgemäßen Instruments und der erfindungsgemäßen Erzeugungseinrichtung sowie der erfindungsgemäßen Vorrichtung ergeben sich aus den Unteransprüchen sowie nachfolgender Beschreibung und den Figuren. Es zeigen schematisiert:
Figur 1 - eine beispielhafte erfindungsgemäße Vorrichtung mit einer erfindungsgemäßen Erzeugungseinrichtung zur Erzeugung eines Aerosols und einem daran angeschlossenen erfindungsgemäßen Instrument mit einem Handhabungsabschnitt,
Figur 2 - ein Ausführungsbeispiel einer Mischeinheit einer erfindungsgemäßen Erzeugungseinrichtung, wie sie beispielsweise in der erfindungsgemäßen Erzeugungseinrichtung gemäß Figur 1 eingesetzt sein kann,
Figur 3a - einen Druckbehälter, welcher einen Flüssigkeitsbehälter bildet, zur Verwendung beispielsweise in einer erfindungsgemäßen Erzeugungseinrichtung gemäß Ausführungsbeispiel aus Figur 1,
Figur 3b - einen Druckbehälter, welcher einen Flüssigkeitsbehälter enthält, zur Verwendung beispielsweise in einer erfindungsgemäßen Erzeugungseinrichtung gemäß Ausführungsbeispiel aus Figur 1,
Figur 4 - ein Ausführungsbeispiel eines erfindungsgemäßen Instruments, wie es beispielsweise in dem Ausführungsbeispiel der erfindungsgemäßen Erzeugungseinrichtung gemäß Figur 1 verwendet werden kann.

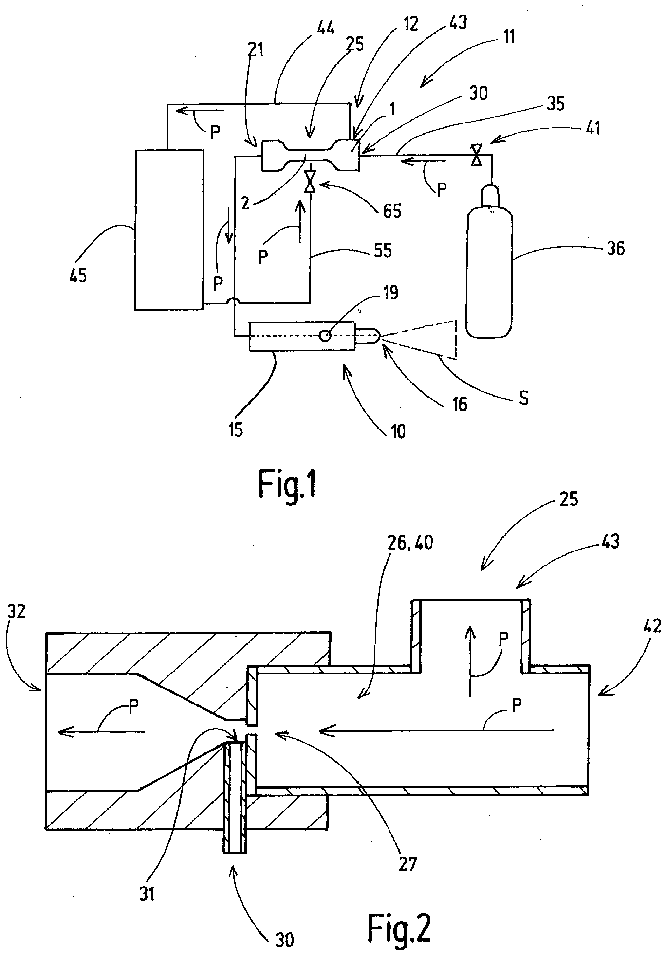

Figur 1 zeigt ein Ausführungsbeispiel einer Vorrichtung mit einem Ausführungsbeispiel einer erfindungsgemäßen Erzeugungseinrichtung zur Erzeugung eines Aerosols und einem Ausführungsbeispiel eines daran angeschlossenen medizinischen Instruments.

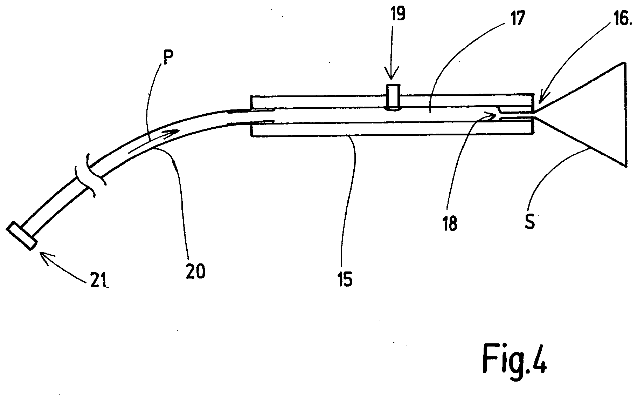

Ein Ausführungsbeispiel des medizinischen Instruments ist in Figur 4 dargestellt. Das medizinische Instrument 10 kann Teil einer Vorrichtung 11 sein, welche zusätzlich eine Erzeugungseinrichtung 12 für Aerosol aufweist. Eine bespielhafte erfindungsgemäße Vorrichtung 11 ist in Figur 1 dargestellt. Das medizinische Instrument 10 weist einen Handhabungsabschnitt 15 zum Handhaben des Instruments 10 beim Durchführen eines medizinischen, insbesondere chirurgischen, Eingriffs auf. Der Handhabungsabschnitt kann auch als Handgriffteil oder Applikator bezeichnet werden. Der Handhabungsabschnitt 15 ist bevorzugt dazu eingerichtet und bestimmt vom Anwender bei der Benutzung des Instruments 10 während des chirurgischen Eingriffs an dem Handhabungsabschnitt 15 ergriffen zu werden, um einen Aerosolauslass 16, welcher am Handhabungsabschnitt 15 angeordnet ist, auszurichten, um in einen gewünschten Bereich gezielt Aerosol abzugeben. In dem Handhabungsabschnitt 15 erstreckt sich ein Kanal 17, welcher an dem Aerosolauslass 16, welcher an dem distalen Ende des Handhabungsabschnitts 15 angeordnet ist, in die Umgebung mündet, aus welchem Aerosolauslass Aerosol als Sprühvollkegel S oder als Strahl ausgegeben werden kann. Der Sprühvollkegel oder Strahl Aerosol kann z.B. zum Präparieren durch Auseinanderdrängen von Gewebestrukturen ohne Schneiden, Befeuchten, Verhindern von thermischer Schädigung durch Kühlen oder zur Sichtverbesserung durch Wegspülen von Flüssigkeiten oder Entfernen von Rauch oder der Verminderung der Entstehung von Rauch verwendet werden. Stromaufwärts des Aerosolauslasses 16 weist der Kanal 17 eine Verengung 18 auf, um eine Düse zum Beschleunigen des Aerosolstroms vor dem Austritt aus dem Aerosolauslass 16 zu bilden. Am Handhabungsabschnitt 15 ist wenigstens ein Bedienelement 19 zum Bedienen des Instruments 10 angeordnet, mit welchem der Aerosolstrom durch den Kanal 17 blockiert oder freigegebenen werden kann. Dies kann beispielsweise durch Abklemmen eines Schlauches oder Röhre mit dem Bedienelement 19 erfolgen, welche den Kanal 17 in dem Handhabungsabschnitt bilden können, um den Aerosolstrom zum Aerosolauslass 16 zu unterbinden. Von der Erzeugungseinrichtung 12 zu dem Handhabungsabschnitt 15 führt eine Aerosolleitung 20, mit welcher der Kanal 17 des Handhabungsabschnitts und damit der Aerosolauslass 16 mit der Erzeugungseinrichtung 12 fluidisch verbunden ist. Bei der Aerosolleitung 12 handelt es sich, zumindest in einem Abschnitt, um eine Schlauchleitung. Erfindungsgemäß wird das Aerosol in der Erzeugungseinrichtung 12 erzeugt, welche am proximalen Ende 21 der Aerosolleitung 20 angeordnet ist.

Die Erzeugungseinrichtung 12 weist bevorzugt eine Mischeinheit 25 auf, welche eine Venturidüse enthält. Ein Beispiel einer Mischeinheit 25, wie sie in dem Ausführungsbeispiel gemäß Figur 1 eingesetzt werden kann, ist in Figur 2 dargestellt. Die Mischeinheit 25 weist einen Kanal 26 auf, welcher eine Verengung 27 aufweist, um die Venturidüse auszubilden. Zum Beaufschlagen des Kanals 26 mit Druckgas stromaufwärts der Verengung 27 weist die Mischeinheit 25 einen Anschluss für Druckgas auf. Stromabwärts der Verengung 27 ist der Kanal 26 mit einem Anschluss 30 der Mischeinheit 25 für ein Fluid verbunden, um den Kanal 26 mit Fluid, insbesondere Flüssigkeit, zu speisen. Die Mischeinheit 25 weist stromabwärts der Stelle 31 des Kanals 26, an welcher das Fluid in den Kanal 26 eintritt, einen Ausgabeanschluss 32 für Aerosol auf. Der Ausgabeanschluss 32 ist mit der Aerosolleitung 20 verbunden, welche zum Handhabungsabschnitt 15 und zum Aerosolauslass 16 des Instruments 10 führt.

Erfindungsgemäß ist die Mischeinheit 25 mittels einer ersten Druckgasleitung 35 mit einer Quelle 36 für Druckgas, insbesondere CO2 verbunden, um die Mischeinheit 25 aus der Quelle 36 mit unter Druck stehendem Gas zu beaufschlagen. Bei der Quelle 36 kann es sich, wie in Figur 1 dargestellt, beispielsweise um einen mobilen Druckgasspeicher, wie beispielsweise eine Gasflasche, oder einen stationären Druckspeicher handeln. Erfindungsgemäß ist zwischen Druckgasspeicher 36 und Mischeinheit 25 keine Pumpe oder Kompressor vorhanden, sondern der Druck des Druckgasspeichers 36 reicht für die Erzeugung des Aerosols aus. Zwischen dem Abschnitt 40 des Kanals 26, welcher stromaufwärts an die Verengung 27 angrenzt und dem Druckgasspeicher 36 ist eine Druckminderungseinrichtung 41 zur Minderung des Drucks aus dem Druckgasspeicher 36 vorhanden. Im dargestellten Ausführungsbeispiel ist dies ein Druckminderer 41, der zwischen dem Druckgasspeicher 36 und dem Anschluss 42 der Mischeinheit 25 für Druckgas angeordnet ist. Die Druckminderungseinrichtung 41 ist dazu eingerichtet, den von der Gasflasche 36 bereitgestellten Gasdruck auf einige Bar in dem Abschnitt 40 des Kanals 26 zu mindern, welcher Abschnitt 40 stromaufwärts an die Verengung 27 angrenzt. Um aus dem Abschnitt 40 des Kanals 26 einen Teil des Gases abzuzweigen, um damit die Flüssigkeit zu der oder in die Mischeinheit 25 zu drücken, weist die Mischeinheit 25 stromaufwärts der Verengung 27 einen Abzweiganschluss 43 zum Abzweigen von Gas in eine zweite Druckgasleitung 44 auf, welche mit einem Behälter 45 fluidisch verbunden ist, welcher mit dem unter Druck stehenden Gas beaufschlagbar ist. Der Behälter 45 ist damit mittels der zweiten Druckgasleitung 44 an denselben Druckgasspeicher 36, wie die Mischeinheit 25 angeschlossen. Der Abzweig 43 zum Beaufschlagen des Behälters 45 aus derselben Quelle 36 für unter Druck stehendes Gas wie die Mischeinheit 25 kann alternativ selbstverständlich auch stromaufwärts der Mischeinheit 25 zwischen Mischeinheit 25 und Quelle 36 angeordnet sein.

Die erfindungsgemäße Vorrichtung 11 weist einen solchen mit dem unter Druck stehenden Gas beaufschlagbaren Behälter 45 (Druckbehälter) auf. Mit dem Gasdruck soll eine Flüssigkeit, insbesondere Kochsalzlösung, welche in dem Druckbehälter 45 enthalten ist, aus dem Behälter 45 zu der Mischeinheit 25 gedrückt werden.

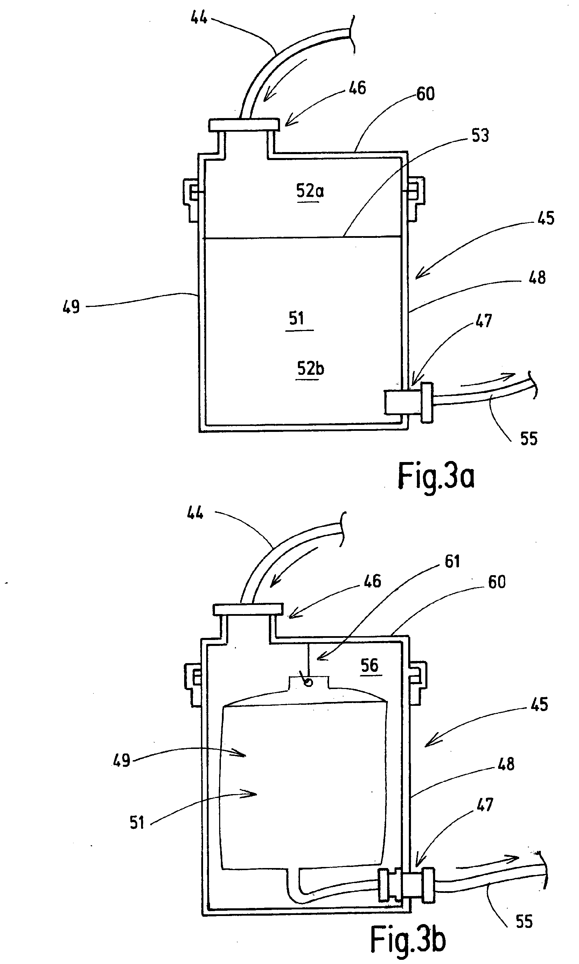

Ein Beispiel einer Ausführungsform des Druckbehälters 45 ist in Figur 3a gezeigt. Der Druckbehälter 45 weist einen Anschluss 46 zum Beaufschlagen des Druckbehälters mit Gas und einen Anschluss 47 zum Ausgeben der Flüssigkeit auf. Der Anschluss 46 zum Beaufschlagen ist mit der zweiten Druckgasleitung 44 verbunden. Die Wandung des Druckbehälters 45 selbst hindert die Flüssigkeit am Auslaufen. Der Druckbehälter 45 gemäß Figur 3a bildet damit einen Flüssigkeitsbehälter 49, welcher bis zu einer Füllhöhe mit Flüssigkeit 51 gefüllt ist. Der Raum 52 oberhalb des Flüssigkeitsspiegels 53 ist über den Anschluss 46 zum Beaufschlagen mit dem unter einem Druck von einigen Bar stehenden Gas beaufschlagbar, so dass das Gas unmittelbar auf den Flüssigkeitsspiegel 53 und damit die Flüssigkeit 51 aus dem Anschluss 47 zum Ausgeben drückt. An dem Anschluss 47 zum Ausgeben ist eine Flüssigkeitsleitung 55 angeschlossen, welche am anderen Ende mit dem Anschluss 30 der Mischeinheit 25 für Fluid verbunden ist.

Ein Beispiel einer zu der Ausführungsform des Druckbehälters 45 gemäß Figur 3a alternativen Ausführungsform ist in Figur 3b dargestellt. Im Unterschied zu der Ausführungsform gemäß Figur 3a bildet der Druckbehälter 45 selbst nicht unmittelbar einen Flüssigkeitsbehälter, sondern der Druckbehälter 45 enthält einen komprimierbaren Flüssigkeitsbehälter 49, beispielsweise einen Beutel 49. Der Raum 56 um den Beutel 49 ist über den Anschluss zum Beaufschlagen 46 mit dem Gas mit einem Druck des Gases von einigen Bar beaufschlagbar, so dass der Beutel 49 komprimiert wird, wobei die Flüssigkeit aus dem Anschluss 47 zum Ausgeben gedrückt wird. Bei dem Beutel 49 kann es sich beispielsweise um einen in der Klinik, in welcher die Vorrichtung 11 eingesetzt wird, verfügbaren Standardbehälter, insbesondere für Kochsalzlösung handeln. Beispielsweise ist der Beutel ein Standardinfusionsbeutel. Bevorzugt ist der Anschluss zum Ausgeben 47 der Flüssigkeit gemäß einem im medizinischen Bereich standardmäßig verwendeten Verbindungssystem ausgebildet. Der Anschluss kann beispielsweise ein Luer-Lock-Anschluss gemäß dem Luer-System sein. Der Flüssigkeitsbehälter 49 kann beispielsweise innerhalb des Druckbehälters 45 an dem Deckel 60 des Druckbehälters 48 mit einer Aufhängvorrichtung 61 aufhängbar sein. Alternativ kann der Druckbehälter 45 dazu eingerichtet sein, dass der Flüssigkeitsbehälter 49 in den Druckbehälter 45 eingelegt wird, welcher so mit der Leitung 35 zwischen der Gasflasche oder dem Druckminderer 41 und dem Abschnitt 40 des Kanals 26 stromaufwärts der Verengung 27 verbunden ist, so dass der Überdruck aus der Gasflasche auf den Flüssigkeitsbehälter 49, insbesondere Beutel 49, wirken kann. Ein Vorteil der Verwendung eines vorbefüllten Flüssigkeitsbehälters 49, beispielsweise eines in der Klinik verfügbaren Standard-, beispielsweise Infusionsbeutels, besteht darin, dass das unmittelbare Befüllen des Druckbehälters 45 als Flüssigkeitsbehälter 49 entfällt. Die Vorkehrungen, um Sterilität der in die Mischeinheit gespeisten Kochsalzlösung zu gewährleisten, sind einfacher, wenn ein Standardbeutel 49 mit steriler isotonischer Kochsalzlösung für die klinische Anwendung verwendet wird.

In zu den Ausführungsbeispielen gemäß Figur 3a und 3b alternativen Ausführungsbeispielen kann beispielsweise eine Membran oder ein Kolben den Raum des Druckbehälters teilen (nicht dargestellt), wobei der eine Raumbereich mit dem unter Druck stehenden Gas beaufschlagt wird, und der andere Raumbereich die Flüssigkeit enthält. Die Membran oder der Kolben wird mittels des Druckgases mit einer Kraft beaufschlagt, um auf eine Flüssigkeitssäule (wie in Figur 3a) unmittelbar oder auf den Flüssigkeitsbehälter (wie in Figur 3b) zu drücken. In den alternativen Ausführungsbeispielen kann der Druckbehälter, wie im Ausführungsbeispiel gemäß Figur 3a, unmittelbar einen Flüssigkeitsbehälter bilden, oder, wie im Ausführungsbeispiel gemäß Figur 3b, einen flexiblen (komprimierbaren) Flüssigkeitsbehälter enthalten.

Zwischen dem Flüssigkeitsbehälter 49 und der Mündung 31 in den Kanal 26 am Anschluss 30 der Mischeinheit 25 für das Fluid kann eine Drosseleinrichtung 65 angeordnet sein, um den Druck in der Flüssigkeitsleitung 55 zwischen dem Flüssigkeitsbehälter und der Mündung 31 in den Kanal 26 zu drosseln. Damit kann die Durchflussrate der Flüssigkeit eingestellt werden. Je Größer der an der Drosseleinrichtung 65 erzeugte Druckabfall ist, desto geringer ist der Durchfluss an Flüssigkeit. Damit kann das Mischungsverhältnis von Gas und Flüssigkeit eingestellt werden. Im Kanalabschnitt 40 der Mischeinheit 25 stromaufwärts der Verengung ist der statische Druck der Gasströmung höher als in der Verengung, hin zu welcher strömend die Strömung beschleunigt wird und der statische Druck infolgedessen absinkt.

Die Erzeugungseinrichtung 12 und/oder die Quelle 36 stützen sich vorzugsweise, beispielsweise über eine oder mehrere Tragvorrichtungen, am Untergrund ab.

Die Vorrichtung 11 arbeitet wie folgt. Die Strömungsrichtungen in den Leitungen und Kanälen sind in den Figuren mit Pfeilen P neben oder in den Leitungen und Kanälen gekennzeichnet:
Der Kanal 26 der Mischeinheit 25 wird mittels der Gasflasche 36 mit Gas, beispielsweise CO2, mit einem Druck von einigen Bar, beispielsweise größer oder gleich 2 Bar, beispielsweise etwa 7 Bar, beaufschlagt. Wird am Handhabungsabschnitt 15 der Durchgang durch die Leitung, welche den Kanal 17 im Handhabungsabschnitt 25 bildet, mittels des Bedienelements 19 freigegeben, strömt das Gas durch den Kanal 17 und die zweite Druckgasleitung 44 in den Druckbehälter 45 und drückt dort die Flüssigkeit aus dem Druckbehälter 45 zu der Mischeinheit 25. Dies kann beispielsweise geschehen, indem der Gasdruck unmittelbar auf den Flüssigkeitsbeutel 49 wirkt und diesen zusammendrückt (gemäß Figur 3b) und damit die Flüssigkeit durch die Flüssigkeitsleitung 55 zu dem Kanal 26 in der Mischeinheit 25, oder indem der Druck unmittelbar auf die Flüssigkeitsoberfläche 53 wirkt (gemäß Ausführungsbeispiel in Figur 3a). In den dargestellten und beschriebenen Ausführungsformen kann auf die Flüssigkeit 51 gedrückt werden, indem der Flüssigkeitsbehälter 49 unter Druck gesetzt wird, um die Flüssigkeit 51 zur Mischeinheit 25 zu fördern. Mit der Ausführungsform gemäß Figur 3b ist sichergestellt, dass die Flüssigkeitsversorgung unabhängig von der Ausrichtung des Beutels 49 bzw. des Druckbehälters 45 ist. Wenn das Gas durch die Venturi-Düse strömt, welche durch die Verengung 27 des Kanals 26 der Mischeinheit 25 gebildet ist, so ist an der engsten Stelle des Kanals 26, welche die Verengung 27 bildet, der dynamische Druck (Staudruck) maximal und der statische Druck minimal. Wenn das Gas durch den Kanal 26 der Mischeinheit 25 strömt, wird das Gas an der Verengung 27 des Kanals beschleunigt, wobei der statische Druck abnimmt und somit unterhalb des Drucks innerhalb des Druckbehälters 45 liegt. Dadurch saugt das Gas Flüssigkeit aus der Flüssigkeitsleitung 55 in den Gasstrom ein, wobei die Flüssigkeit zu Flüssigkeitströpfchen zerstäubt wird, welche von dem Gasstrom mitgerissen werden. Der stromaufwärts an die Verengung 27 angrenzende Abschnitt 40 des Kanals 26 der Mischeinheit 25 ist in dem Ausführungsbeispiel gemäß Figur 3a mit dem unteren Raum 50b des Behälters 45 verbunden, den die Kochsalzlösung einnimmt. Der Unterschied zwischen dem statischen Druck in der Verengung 27 und dem Druck in dem Druckbehälter 45 führt dazu, dass die Kochsalzlösung an der Verengung 27 stromabwärts von dieser in die Gasströmung befördert wird und sich dort mit der Gasströmung vermischt. Der Druck in dem Druckbehälter 45 hängt von dem Druck in dem Abschnitt 40 des Kanals 26 ab, welcher stromaufwärts an die Verengung 27 angrenzt, da der Druckbehälter 45 über die zweite Druckgasleitung 44 an den Abschnitt 40 angeschlossen ist.

Das so erzeugte Aerosol tritt aus dem Aerosolausgang der Mischeinheit 25 in die Aerosolleitung 20 ein und wird durch den Kanal 17 in dem Handhabungsabschnitt 15 zum Aerosolauslass 16 des Instruments 10 am Handhabungsabschnitt 15 geleitet, um dort ausgegeben zu werden. In der Düse stromaufwärts des Aerosolauslasses 16 am Handhabungsabschnitt 15, welche durch die Verengung 18 gebildet wird, wird der Aerosolstrom zuvor mittels der Düse beschleunigt.

Wenn, wie in den beschriebenen Ausführungsformen, die Mischung des Aerosols am proximalen Ende 21 des Instruments 10 erfolgt, kann die Weiterleitung des Gas-Wassergemisches zum distalen Ende des Handhabungsabschnitts 15 (Aerosolapplikators) durch nur eine Aerosolleitung 20 erfolgen, so dass auf eine weitere Leitung verzichtet werden kann. Die Mischeinheit 25 kann beispielsweise in einem Gerät angeordnet sein, an welchem das Instrument 10 angeschlossen ist. Die geräteseitige Mischeinheit 25, in welcher das Gas und die Flüssigkeit zusammengeführt werden, kann beispielsweise Y-förmig sein.

Die Förderung des Flüssigkeitsstroms kann dabei vorzugsweise mittels einer passiven Fördereinheit erfolgen, also ohne elektrisch betriebene Pumpe. Bevorzugt ist zwischen dem Flüssigkeitsbehälter 49 und der Stelle 31, an welcher die Flüssigkeit in den Kanal 26 in der Mischeinheit 25 eintritt, keine elektrisch betriebene Pumpe angeordnet, welche eingerichtet und bestimmt wäre, Flüssigkeit in den Kanal 26 der Mischeinheit 25 zu fördern. Dazu wird gemäß Ausführungsformen der Erfindung der Druck eines Druckgasspeichers 36, wie beispielsweise eine Gasflasche genutzt, um die Mischeinheit 25, welche zusätzlich bevorzugt eine Strahlpumpe bilden kann, zu speisen und andererseits die Flüssigkeit aus dem Flüssigkeitsbehälter 49 zu der Mischeinheit 25 zu drücken. Gegenüber einer Vorrichtung, welche eine aktive Fördereinheit für die Flüssigkeit, wie z.B. eine Spritzenpumpe oder einer Rollenpumpe, aufweist, bietet die erfindungsgemäße Vorrichtung 11, in Ausführungsformen, in denen auf eine solche Pumpe zwischen dem Flüssigkeitsbehälter 49 und der Mischeinheit 25 verzichtet wird, den Vorteil, dass die aktive Fördereinheit in der Vorrichtung eingespart werden kann. Die erfindungsgemäße Vorrichtung 11 ist dadurch vergleichsweise wenig aufwändig in der Herstellung und Wartung und daher vergleichsweise günstig. Außerdem ist der Massenstrom an Flüssigkeit von dem angelegten Druck in der ersten Druckgasleitung 35 abhängig. Damit hängt auch die Menge der angesaugten Flüssigkeit von dem Druck in der ersten Druckgasleitung 35 ab. Die vorgeschlagene Förderung bietet daher eine Selbstjustage. Je stärker die von der Mischeinheit 25 mit der Verengung 27 gebildete Strahlpumpe an der Flüssigkeitsleitung 55 saugt, desto stärker wird durch die Druckbeaufschlagung des Druckbehälters 45 Flüssigkeit aus dem Flüssigkeitsbehälter 49 in die Flüssigkeitsleitung 55 gedrückt.

Erfindungsgemäß wird ein medizinisches Instrument 10 mit einem Aerosolauslass 16 angegeben, welches über eine Aerosolleitung 20 fluidisch mit einer Erzeugungseinrichtung 12 verbunden ist. Die Erzeugungseinrichtung 12 ist dazu eingerichtet, Aerosol zur Abgabe mittels des medizinischen Instruments 10 zu erzeugen. Es wird zudem eine Erzeugungseinrichtung 12 angegeben, welche in Ausführungsformen der Erzeugungseinrichtung 12 beispielsweise in einer Vorrichtung 11 mit einem erfindungsgemäßen Instrument 10 eingesetzt werden kann. Die Erzeugungseinrichtung 12 weist eine Mischeinheit 25 auf, um eine Flüssigkeit und ein Gas miteinander zu mischen, welche Mischeinheit 25 an einen Flüssigkeitsbehälter 49 zur Versorgung der Mischeinheit 25 mit Flüssigkeit angeschlossen ist, wobei die Erzeugungseinrichtung 12 dazu eingerichtet ist, den Gasdruck eines unter Druck stehenden Gases zu nutzen, die Flüssigkeit mit Druck zu beaufschlagen, um die Flüssigkeit aus dem Flüssigkeitsbehälter 49 zu der Mischeinheit 25 zu drücken.

**Bezugszeichenliste:**

| | |
|---|---|
| 10 | Medizinisches Instrument |
| 11 | Vorrichtung |
| 12 | Erzeugungseinrichtung |
| 15 | Handhabungsabschnitt |
| 16 | Aerosolauslass |
| 17 | Kanal |
| 18 | Verengung |
| 19 | Bedienelement |
| 20 | Aerosolleitung |
| 21 | Proximales Ende |
| 25 | Mischeinheit |
| 26 | Kanal der Mischeinheit |
| 27 | Verengung des Kanals der Mischeinheit |
| 30 | Anschluss |
| 31 | Stelle/Mündung |
| 32 | Ausgabeanschluss |
| 35 | Erste Druckgasleitung |
| 36 | Quelle/Druckgasspeicher/Gasflasche |
| 40 | Abschnitt |
| 41 | Druckminderer |
| 42 | Anschluss |
| 43 | Abzweiganschluss/Abzweig |
| 44 | Zweite Druckgasleitung |
| 45 | Behälter/Druckbehälter |
| 46 | Anschluss |
| 47 | Anschluss |
| 48 | Wandung des Druckbehälters |
| 49 | Flüssigkeitsbehälter/Beutel |
| | |
| 51 | Flüssigkeit |
| 52a | Raum |
| 52b | Raum |
| 53 | Flüssigkeitsspiegel |
| 55 | Flüssigkeitsleitung |
| 56 | Raum |
| 60 | Deckel |
| 61 | Aufhängvorrichtung |
| 65 | Drosseleinrichtung |
| S | Sprühvollkegel Aerosol |
| P | Pfeil |

## Patentansprüche

1. Einrichtung, umfassend ein medizinisches Instrument (10) und eine Erzeugungseinrichtung (12),
wobei das Instrument (10) einen Aerosolauslass (16) aufweist und wobei das Instrument (10) mittels einer Aerosolleitung (20) mit der Erzeugungseinrichtung (12) fluidisch verbunden ist, welche Erzeugungseinrichtung (12) dazu eingerichtet ist, Aerosol zur Abgabe mittels des medizinischen Instruments (10) durch Zerstäubung einer Flüssigkeit mittels eines Gasstroms aus einer Druckquelle (36) zu erzeugen,
wobei die Erzeugungseinrichtung (12) eine Mischeinheit (25) aufweist, um eine Flüssigkeit und ein Gas miteinander zu mischen, welche Mischeinheit (25) an einen Flüssigkeitsbehälter (49) zur Versorgung der Mischeinheit (25) mit Flüssigkeit angeschlossen ist,
**dadurch gekennzeichnet, dass**
die Erzeugungseinrichtung (12) dazu eingerichtet ist, den Gasdruck des unter Druck stehenden Gases aus derselben Druckquelle (36) zu nutzen, die Flüssigkeit mit Druck zu beaufschlagen, um die Flüssigkeit aus dem Flüssigkeitsbehälter (49) zu der Mischeinheit (25) zu drücken.

2. Einrichtung nach Anspruch 1, wobei sich die Erzeugungseinrichtung (12) bei der medizinischen Anwendung des Instruments (10) am Untergrund abstützt.

3. Einrichtung nach einem der vorstehenden Ansprüche, wobei die Länge der Aerosolleitung (20) zwischen der Erzeugungseinrichtung (12) und dem Aerosolauslass (16) oder zwischen der Erzeugungseinrichtung (12) und dem Instrument (16) wenigstens 1 Meter, bevorzugt wenigstens 2 Meter beträgt.

4. Einrichtung nach einem der vorstehenden Ansprüche, wobei die Erzeugungseinrichtung (12) derart eingerichtet ist, dass Flüssigkeit ohne elektrisch betriebene Pumpe zu der Erzeugungseinrichtung (12) gefördert wird.

5. Einrichtung nach einem der vorstehenden Ansprüche, wobei das Instrument und/oder die Erzeugungseinrichtung (12) dazu eingerichtet sind, das Aerosol ohne elektrisch betriebene Pumpe zu dem Aerosolauslass (16) zu fördern.

6. Einrichtung nach Anspruch 1, wobei die Erzeugungseinrichtung (12) an eine Gasflasche (36) oder ein stationäres Druckgasnetz als Quelle (36) für das unter Druck stehende Gas angeschlossen ist.

7. Einrichtung nach Anspruch 5 oder 6, wobei die Flüssigkeit in einem Druckbehälter (45) enthalten ist, welcher mit dem unter Druck stehenden Gas beaufschlagbar ist.

8. Einrichtung nach einem der Ansprüche 1 bis 7, wobei die Erzeugungseinrichtung (12) derart ausgebildet ist, dass der Flüssigkeitsbehälter (49) von außen mit Druck beaufschlagt wird, um die Flüssigkeit zu der Mischeinheit (25) zu fördern.

9. Einrichtung nach einem der Ansprüche 1 bis 8, wobei der Flüssigkeitsbehälter (49) dazu eingerichtet ist, komprimiert zu werden.

10. Einrichtung nach Anspruch 9, wobei der Flüssigkeitsbehälter (49) ein Beutel, insbesondere ein Standardbeutel für Kochsalzlösung ist, wie er in einer Klinik verwendet wird.

11. Einrichtung nach einem der Ansprüche 9 bis 10, wobei der Flüssigkeitsbehälter (49) in einem Druckbehälter (45) angeordnet ist, wobei der Druckbehälter (45) mit Druckgas beaufschlagbar ist.

12. Einrichtung nach einem der Ansprüche 9 bis 11, wobei der Gasdruck des Gases unmittelbar auf den Flüssigkeitsbehälter (49) wirkt.

13. Einrichtung nach einem der vorstehenden Ansprüche, wobei die Mischeinheit (25) einen Kanal (26) mit einer Verengung (27) aufweist, wobei der Kanal (26) stromaufwärts der Verengung (27) an die Druckquelle (36) angeschlossen ist, wobei der Flüssigkeitsbehälter (49) an der Verengung (27) und/oder stromabwärts der Verengung (27) an den Kanal (26) angeschlossen ist, um mittels des Druckabfalls auf Grund der Verengung Flüssigkeit aus dem Flüssigkeitsbehälter (49) in den Kanal (26) zu fördern.

14. Einrichtung nach Anspruch 13, wobei der Kanal (26) stromaufwärts der Verengung (27) an die Druckquelle (36) angeschlossen ist, welche die Erzeugungseinrichtung (12) nutzt, um die Flüssigkeit mit Druck zu beaufschlagen.

15. Einrichtung nach Anspruch 14, wobei eine an die Druckquelle (36) angeschlossene Leitung (35, 26) zwischen der Verengung (27) und der Druckquelle (36) verzweigt, um aus der Leitung (35, 26) sowohl die Verengung (27) mit Gas zu speisen als auch das Gas zum Beaufschlagen der Flüssigkeit mit Druck bereitzustellen.

## Claims

1. Device comprising a medical instrument (10) and a creation device (12), wherein the instrument (10) has an aerosol outlet (16) and wherein the instrument (10) is fluidically connected to the creation device (12) by means of an aerosol line (20), which creation device (12) is configured to create an aerosol for output by means of the medical instrument (10) by atomisation of a liquid by means of a gas stream from a pressure source (36),
wherein the creation device (12) comprises a mixing unit (25) for mixing a liquid and gas together, which mixing unit (25) is connected to a liquid container (49) for supplying the mixing unit (25) with liquid,
**characterised in that** the creation device (12) is configured to use the gas pressure of the pressurised gas from the same pressure source (36) to pressurise the liquid, in order to press the liquid from the liquid container (49) to the mixing unit (25).

2. Device according to claim 1, wherein the creation device (12) rests on the floor during medical use of the instrument (10).

3. Device according to any preceding claims, wherein the length of the aerosol line (20) between the creation device (12) and the aerosol outlet (16), or between the creation device (12) and the instrument (16), is at least 1 metre, preferably at least 2 metres.

4. Device according to any of the preceding claims, wherein the creation device (12) is configured such that the liquid is conveyed to the creation device (12) without an electrically operated pump.

5. Device according to any of the preceding claims, wherein the instrument and/or the creation device (12) are configured to convey the aerosol to the aerosol outlet (16) without an electrically operated pump.

6. Device according to claim 1, wherein the creation device (12) is connected to a gas cylinder (36) or to a stationary compressed gas network as a source (36) for the pressurised gas.

7. Device according to claim 5 or 6, wherein the liquid is contained in the pressure vessel (45) which can be loaded with pressurised gas.

8. Device according to any of claims 1 to 7, wherein the creation device (12) is configured such that the liquid container (49) can be pressurised from the outside in order to convey the liquid to the mixing unit (25).

9. Device according to any of claims 1 to 8, wherein the liquid container (49) is configured to be compressed.

10. Device according to claim 9, wherein the liquid container (49) is a bag, in particular a standard bag for saline solution as used in hospitals.

11. Device according to one of claims 9 or 10, wherein the liquid container (49) is arranged in a pressure vessel (45), wherein the pressure vessel (45) can be loaded with compressed gas.

12. Device according to any of claims 9 to 11, wherein the gas pressure of the gas acts directly on the liquid container (49).

13. Device according to any of the preceding claims, wherein the mixing unit (25) has a channel (26) with a constriction (27), wherein the channel (26) is connected to the pressure source (36) upstream of the constriction (27), wherein the liquid container (49) is connected to the constriction (27) and/or to the channel (26) downstream of the constriction (27) in order to convey liquid from the liquid container (49) into the channel (26) by means of the pressure fall due to the constriction.

14. Device according to claim 13, wherein the channel (26) is connected to the pressure source (36), which the creation device (12) uses to pressurise the liquid, upstream of the constriction (27).

15. Device according to claim 14, wherein a line (35, 26) connected to the pressure source (36) branches between the constriction (27) and the pressure source (36) in order both to feed gas from the line (35, 26) to the constriction and to provide the gas for pressurising the liquid.

## Revendications

1. Dispositif, comprenant un instrument médical (10) et un dispositif de génération (12),
dans lequel l'instrument (10) présente une sortie d'aérosol (16), et dans lequel l'instrument (10) est relié au moyen d'une conduite d'aérosol (20) au dispositif de génération (12) en vue d'une communication de fluide, ledit dispositif de génération (12) étant conçu pour générer un aérosol destiné à être délivré au moyen de l'instrument médical (10), par atomisation d'un liquide à l'aide d'un flux de gaz provenant d'une source de pression (36),
dans lequel le dispositif de génération (12) présente une unité de mélange (25) destinée à mélanger un liquide et un gaz, ladite unité de mélange (25) étant raccordée à un réservoir de liquide (49) destiné à alimenter l'unité de mélange (25) avec du liquide,
**caractérisé en ce que**
le dispositif de génération (12) est conçu pour utiliser la pression gazeuse du gaz sous pression provenant de la même source de pression (36), pour appliquer une pression au liquide afin de refouler le liquide du réservoir de liquide (49) vers l'unité de mélange (25).

2. Dispositif selon la revendication 1, dans lequel le dispositif de génération (12) prend appui sur le support lors de l'application médicale de l'instrument (10).

3. Dispositif selon l'une des revendications précédentes, dans lequel la longueur de la conduite d'aérosol (20) entre le dispositif de génération (12) et la sortie d'aérosol (16) ou entre le dispositif de génération (12) et l'instrument (16) est au moins de 1 mètre, de préférence au moins de 2 mètres.

4. Dispositif selon l'une des revendications précédentes, dans lequel le dispositif de génération (12) est conçu de manière à ce que le liquide soit transporté jusqu'au dispositif de génération (12) sans pompe à commande électrique.

5. Dispositif selon l'une des revendications précédentes, dans lequel l'instrument et/ou le dispositif de génération (12) sont conçus pour transporter l'aérosol jusqu'à la sortie d'aérosol (16) sans pompe à commande électrique.

6. Dispositif selon la revendication 1, dans lequel le dispositif de génération (12) est raccordé à une bouteille de gaz (36) ou un réseau de gaz comprimé stationnaire en tant que source (36) pour le gaz sous pression.

7. Dispositif selon la revendication 5 ou 6, dans lequel le liquide est contenu dans un réservoir sous pression (45) auquel peut être appliqué le gaz sous pression.

8. Dispositif selon l'une des revendications 1 à 7, dans lequel le dispositif de génération (12) est conçu de manière à ce qu'une pression soit appliquée de l'extérieur au réservoir de liquide (49), afin de transporter le liquide jusqu'à l'unité de mélange (25).

9. Dispositif selon l'une des revendications 1 à 8, dans lequel le réservoir de liquide (49) est conçu pour être comprimé.

10. Dispositif selon la revendication 9, dans lequel le réservoir de liquide (49) est une poche, en particulier une poche standard pour une solution saline, telle qu'elle est utilisée dans une clinique.

11. Dispositif selon l'une des revendications 9 à 10, dans lequel le réservoir de liquide (49) est placé dans un réservoir sous pression (45), le réservoir sous pression (45) pouvant recevoir du gaz comprimé.

12. Dispositif selon l'une des revendications 9 à 11, dans lequel la pression du gaz agit directement sur le réservoir de liquide (49).

13. Dispositif selon l'une des revendications précédentes, dans lequel l'unité de mélange (25) présente un conduit (26) comportant un rétrécissement (27), le conduit (26) étant raccordé à la source de pression (36), en amont du rétrécissement (27), et le réservoir de liquide (49) étant raccordé au conduit (26), à l'endroit du rétrécissement (27) et/ou en aval du rétrécissement (27), afin de transporter du liquide du réservoir de liquide (49) dans le conduit (26), par le biais de la baisse de pression due au rétrécissement.

14. Dispositif selon la revendication 13, dans lequel le conduit (26), en amont du rétrécissement (27), est raccordé à la source de pression (36) qu'utilise le dispositif de génération (12) pour appliquer une pression au liquide.

15. Dispositif selon la revendication 14, dans lequel une conduite (35, 26) raccordée à la source de pression (36) se ramifie entre le rétrécissement (27) et la source de pression (36) pour assurer, à partir de la conduite (35, 26), à la fois l'alimentation en gaz du rétrécissement (27) et la distribution du gaz destiné à appliquer une pression au liquide.
